(19) **Europäisches Patentamt / European Patent Office / Office européen des brevets**

(11) **EP 3 103 433 A1**

(12) # DEMANDE DE BREVET EUROPEEN

(43) Date de publication:
**14.12.2016 Bulletin 2016/50**

(51) Int Cl.:
***A61K 8/29*** *(2006.01)*     ***A61K 8/41*** *(2006.01)*
***A61K 8/49*** *(2006.01)*     ***A61Q 17/04*** *(2006.01)*

(21) Numéro de dépôt: **16172282.2**

(22) Date de dépôt: **31.05.2016**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD**

(30) Priorité: **10.06.2015 FR 1555290**

(71) Demandeur: **ACM**
**92110 Clichy (FR)**

(72) Inventeur: **LEVEILLE NIZEROLLE, Thierry**
**92100 BOULOGNE BILLANCOURT (FR)**

(74) Mandataire: **Novagraaf Technologies**
**Bâtiment O2**
**2, rue Sarah Bernhardt**
**CS90017**
**92665 Asnières-sur-Seine Cedex (FR)**

(54) **COMPOSITION COSMETIQUE ANTI-UV ET LUMIERE VISIBLE DE HAUTE ENERGIE**

(57)     La présente invention se rapporte à une composition cosmétique ou dermatologique contre les rayonnements UV et la lumière visible de haute énergie comprenant du diéthylamino hydoxybenzoyl hexyl benzoate, du méthylene bis-benzotriazolyl tetramethylbutylphenol, de l'éthylhexyl triazone, du bis-ethylhexyloxyphenol methoxyphenyl triazine et du dioxide de titane ($TiO_2$).

La présente invention se rapporte également à un procédé de traitement cosmétique non thérapeutique contre les rayonnements UV et la lumière visible de haute énergie.

La présente invention trouve une application notamment dans les domaines cosmétiques et dermatologiques.

EP 3 103 433 A1

**Description**

**Domaine technique**

La présente invention se rapporte à une composition cosmétique ou dermatologique contre les rayonnements UV et la lumière visible de haute énergie comprenant du diéthylamino hydroxybenzoyl hexyl benzoate, du méthylene bis-benzotriazolyl tetramethylbutylphenol, de l'éthylhexyl triazone, du bis-ethylhexyloxyphenol methoxyphenyl triazine et du dioxide de titane ($TiO_2$).

La présente invention se rapporte également à un procédé de traitement cosmétique non thérapeutique contre les rayonnements UV-Visible comprenant l'application sur la peau d'une composition cosmétique comprenant du diéthylamino hydroxybenzoyl hexyl benzoate, du méthylene bis-benzotriazolyl tetramethylbutylphenol, de l'éthylhexyl triazone, du bis-ethylhexyloxuphenol methoxyphenyl triazine et du dioxide de titane ($TiO_2$).

La présente invention se rapporte également à l'utilisation d'une composition cosmétique comprenant du diéthylamino hydroxybenzoyl hexyl benzoate, du méthylene bis-benzotriazolyl tetramethylbutylphenol, de l'éthylhexyl triazone, du bis-ethylhexyloxuphenol methoxyphenyl triazine et du dioxide de titane ($TiO_2$).pour la protection de la peau contre les rayonnements UltraViolet (U.V.) et la lumière visible de haute énergie

La présente invention trouve une application notamment dans les domaines cosmétiques et dermatologiques.

Dans la description ci-dessous, les références entre crochets ([ ]) renvoient à la liste des références présentées à la fin du texte.

**Etat de la technique**

L'épiderme est une structure complexe dont les composants cellulaires principaux sont les kératinocytes à l'origine de la production de kératine et de kératohyaline, les cellules de Langerhans qui participent à la défense immunitaire et enfin les mélanocytes dont la fonction principale est la production du pigment mélanique.

La pigmentation de la peau varie d'une personne à l'autre et peut être modifiée en fonction, par exemple de l'âge, de l'exposition de la peau aux rayonnements du soleil, à des changements hormonaux etc.

En particulier, avec le vieillissement notamment la peau peut présenter des taches de vieillesse qui peuvent être dues à une exposition au soleil. Par ailleurs, une variation non uniforme de la pigmentation de la peau peut être due notamment à des modifications hormonales, par exemple lors de la grossesse et provoquant des taches de grossesse ou mélasma. Ces taches peuvent être disgracieuses et à l'origine d'un sentiment de mal-être pour la personne concernée.

L'exposition au soleil est connue dans l'état de la technique pour provoquer et/ou aggraver l'apparition de telles taches/pigmentation irrégulière de la peau. En outre récemment, des publications scientifiques ont démontré que la lumière visible bleue et violette stimulait également la pigmentation de la peau en particulier dans le cadre du mélasma.

Afin d'éviter l'apparition de ces modifications pigmentaires, en particulier d'éviter une stimulation de la pigmentation de la peau, il existe dans l'état de la technique des compositions cosmétiques pour protéger sa peau des rayons UVA et UVB du soleil, avec un indice élevé de protection.

Toutefois, ces compositions, notamment pour des personnes atteintes d'hyperpigmentation épidermiques, de type taches brunes ou mélasma (masque de grossesse), les compositions cosmétique ou écran anti-UV sont insuffisants, notamment car ils ne filtrent pas la lumière bleue ou violette qui sont pourtant pigmentogènes.

Il existe donc un réel besoin dans l'état de la technique de trouver une composition permettant à la fois de filtrer et/ou protéger la peau des rayonnements UVA et UVB mais aussi des lumières bleue et violette pigmentogènes.

Il existe également dans l'état de la technique des écrans teintés apportant, lorsqu'ils sont appliqués de manière homogène, une protection potentielle contre la lumière visible. Toutefois, ces écrans par leur teinte sont affichants et ne sont pas adaptés aux souhaits de nombreux sujets présentant sur le visage des masques de grossesses car ils ne permettent pas d'obtenir un effet esthétique et donc contraire à l'effet escompté.

Il existe donc un réel besoin dans l'état de la technique de trouver une composition transparente permettant à la fois de filtrer et/ou protéger la peau des rayonnements UVA et UVB mais aussi des lumières bleue et violette pigmentogènes et présentant, après application, un aspect esthétique.

Il existe également dans l'état de la technique des écrans transparents permettant à la fois de filtrer et/ou protéger la peau des rayonnements UVA et UVB et ne provoquant pas de coloration inesthétique de la peau. Toutefois, ces compositions ou écrans, ne permettent pas de filtrer la lumière bleue ou violette qui sont pourtant pigmentogènes et donc susceptible d'aggraver l'hyperpigmentation.

Il existe donc un réel besoin dans l'état de la technique de trouver une composition palliant ces défauts, inconvénients et obstacles de l'art antérieur, en particulier une composition à la fois de filtrer et/ou protéger la peau des rayonnements UVA et UVB mais aussi des lumières bleue et violette pigmentogènes.

**Description de l'invention**

La présente invention a précisément pour but de répondre aux besoins de l'art en fournissant une composition cosmétique ou dermatologique contre les rayonnements UV et la lumière de haute énergie comprenant du diéthylamino hydoxybenzoyl hexyl benzoate, du méthylene bis-benzotriazolyl tetramethylbutylphenol, de l'éthylhexyl triazone, du bis-ethylhexyloxyphenol methoxyphenyl triazine et du dioxide de titane ($TiO_2$).

Les inventeurs ont démontrés de manière surprenante que, outre les rayonnements UVA, UVB, la lumière visible en particulier de haute énergie par exemple la lumière bleue et violette est pigmentogène.

Les travaux réalisés par les inventeurs dans le cadre de l'invention ont permis de mettre en évidence, de manière surprenante, que la composition selon l'invention permet avantageusement de protéger la peau des rayonnements UVA, UVB et également de la lumière visible, en particulier la lumière de haute énergie.

En particulier, les travaux réalisés par les inventeurs dans le cadre de l'invention ont permis de mettre en évidence, de manière surprenante, que la composition selon l'invention permet de filtrer les rayons UVA, UVB et également la lumière visible de haute énergie, par exemple la lumière violette et bleue.

De plus, les travaux réalisés par les inventeurs dans le cadre de l'invention ont permis de mettre en évidence, de manière surprenante, que la composition selon l'invention permet de filtrer/d'empêcher le passage/bloquer les rayons UVA, UVB et également la lumière visible de haute énergie, par exemple la lumière violette et bleue correspondant à une diminution de la transmission/du passage des rayonnements de 80% allant jusqu'à une absence totale de transmission/du passage des rayonnements jusqu'à la peau.

En outre, les inventeurs ont démontrés de manière surprenante que la composition selon l'invention permet lors de son utilisation d'obtenir une protection de la peau contre les rayons UVA, UVB et la lumière visible bleu et violette. Ainsi les inventeurs ont démontré que l'utilisation de la composition permet d'obtenir une protection parfaite de la peau contre les rayonnements et la lumière pigmentogènes.

Dans la présente par «rayonnements UV et la lumière de haute énergie» on entend les rayonnements Ultra-Violet (UV) comprenant les UV-B avec une longueur d'ondes de 280-315 nm, les UV-A avec une longueur d'ondes de 315-400nm, et la lumière visible de haute énergie à savoir la lumière visible avec une longueur d'onde de 380-483nm comprenant le champs chromatique violet avec une longueur d'onde de 380-449 nm, violet-bleu avec une longueur d'onde de 449-466nm, bleu-violet avec une longueur d'onde de 466-478 nm et Bleu avec une longueur d'onde de 478-483nm.

En d'autres termes, dans la présente par «rayonnements UV et la lumière de haute énergie» on entend les rayonnements Ultra-Violet (UV) comprenant les UV-B avec une longueur d'ondes compris de 280 à 315 nm, les UV-A avec une longueur d'ondes compris de 315 à 400nm, et la lumière visible de haute énergie à savoir la lumière visible avec une longueur d'onde compris de 380 à 483nm comprenant le champs chromatique violet avec une longueur d'onde compris de 380 à 449 nm, violet-bleu avec une longueur d'onde compris de 449 à 466nm, bleu-violet avec une longueur d'onde compris de 466 à 478 nm et Bleu avec une longueur d'onde compris de 478 à 483nm.

Selon l'invention, le diéthylamino hydoxybenzoyl hexyl benzoate peut être tout diéthylamino hydoxybenzoyl hexyl benzoate connu de l'homme du métier et/ou disponible dans le commerce adapté à une utilisation dans une composition cosmétique et/ou dermatologique. Il peut s'agir par exemple du diéthylamino hydoxybenzoyl hexyl benzoate commercialisé sous la référence Uvinul A plus granular par la société BASF.

Selon l'invention le méthylene bis-benzotriazolyl tetramethylbutylphenol peut être tout méthylene bis-benzotriazolyl tetramethylbutylphenol connu de l'homme du métier et/ou disponible dans le commerce adapté à une utilisation dans une composition cosmétique et/ou dermatologique. Il peut s'agir par exemple du méthylene bis-benzotriazolyl tetramethylbutylphenol commercialisé sous la référence Tinosorb M par la société BASF.

Selon l'invention l'éthylhexyl triazone peut être tout éthylhexyl triazone connu de l'homme du métier et/ou disponible dans le commerce adapté à une utilisation dans une composition cosmétique et/ou dermatologique. Il peut s'agir par exemple de l'éthylhexyl triazone commercialisé sous la référence Uvinul T150 par la société BASF.

Selon l'invention le bis-ethylhexyloxuphenol methoxyphenyl triazine peut être tout bis-ethylhexyloxuphenol methoxyphenyl triazine connu de l'homme du métier et/ou disponible dans le commerce adapté à une utilisation dans une composition cosmétique et/ou dermatologique. Il peut s'agir par exemple du bis-ethylhexyloxuphenol methoxyphenyl triazine commercialisé sous la référence Tinosorb S par la société BASF.

Selon l'invention, le dioxide de titane ($TiO_2$) peut être tout dioxide de titane ($TiO_2$) connu de l'homme du métier et/ou disponible dans le commerce adapté à une utilisation dans une composition cosmétique et/ou dermatologique. Il peut s'agir par exemple du dioxide de titane ($TiO_2$) commercialisé sous la référence SOLAVEIL XT 300-LQ-WD par la société CRODA.

Selon l'invention, le diéthylamino hydoxybenzoyl hexyl benzoate peut être présent dans la composition par exemple à une concentration de 5 à 10 % en poids par rapport au poids total de ladite composition, de préférence de 7 % à 9 % en poids par rapport au poids total de ladite composition.

Selon l'invention, le Bis-ethylhexyloxyphénol methoxyphenyl Triazine peut être présent dans la composition par exemple à une concentration de 5% à 10% en poids par rapport au poids total de la composition, de préférence de 4% à 6% en poids par rapport au poids total de ladite composition.

Selon l'invention, l'éthylhexyl triazone peut être présent dans la composition par exemple à une concentration de 1% à 5% en poids par rapport au poids total de la composition, de 3% à 5%, de préférence de 4 à 5% en poids par rapport au poids total de ladite composition.

Selon l'invention, le méthylène bis-benzotraizolyl peut être présent dans la composition par exemple à une concentration de 1% à 5% en poids par rapport au poids total de la composition, de 4% à 5% de préférence de 4 à 4,5% en poids par rapport au poids total de ladite composition.

Selon l'invention, le dioxide de titane peut être présent dans la composition par exemple à une concentration de 1% à 5% en poids par rapport au poids total de la composition, de préférence de 2,5% à 4%, de préférence de 2,5 à 3,5% en poids par rapport au poids total de ladite composition.

Selon l'invention, la composition peut comprendre en outre au moins un, au moins deux, au moins trois, au moins quatre émulgateur choisis dans le groupe comprenant glycéryl stéarate, le Peg 100 stearate, potassium cetyl phosphate, cocoglucoside, coconut alcohol ou un mélange de ceux-ci.

Selon l'invention, ledit au moins un émulgateur peut être présent dans la composition par exemple à une concentration de 4 à 8 % en poids par rapport au poids total de la composition, de préférence de 6 à 7 % en poids par rapport au poids total de la composition.

Selon l'invention, la composition peut comprendre en outre au moins un solubilisant. Il peut s'agir de préférence du Diisopropyl Sebacate.

Selon l'invention, ledit au moins un solubilisant peut être présent dans la composition par exemple à une concentration de 10 à 20 % en poids par rapport au poids total de la composition, de préférence de 12 à 17 % en poids par rapport au poids total de la composition.

Selon l'invention, la composition cosmétique peut être sous toute forme connue de l'homme du métier dans les domaines de la cosmétique et de la dermatologie, sans aucune restriction galénique particulière.

La composition cosmétique ou dermatologique peut être sous toute forme galénique connue de l'homme du métier, par exemple sous la forme d'un onguent, d'un sérum, d'une crème, d'une huile, d'un lait, d'une pommade, d'une poudre, d'un stick, d'une solution, d'un gel, d'un spray, d'une lotion ou d'une suspension. Il peut s'agir par exemple d'une crème, d'un lait, d'un gel, d'une lotion, d'un onguent.

Selon l'invention, quel(s) que soi(en)t l'extrait ou le mélange d'extraits utilisés, la composition cosmétique ou dermatologique de la présente invention peut comprendre en outre au moins un agent actif additionnel choisi, par exemple parmi un agent anti-âge, un agent hydratant, un agent apaisant et/ou un mélange de ces agents. Ces agents peuvent être ceux couramment utilisés dans les produits cosmétiques et/ou dermatologiques. Ce ou ces agent(s) peuvent être utilisé(s) dans la composition de la présente invention par exemple aux concentrations usuellement utilisées et connues de l'homme du métier dans les compositions cosmétiques ou dermatologiques

La composition cosmétique selon l'invention peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des agents épaississants minéraux, des parfums, des conservateurs, des vitamines et des provitamines, des protéines, des agents séquestrants, des agents alcanisants, des agents acidifiants, des agents réducteurs, des agents oxydants, des charges minérales, des colorants naturels ou tout autre adjuvant pouvant être cité dans le dictionnaire INCI (International Nomenclature Cosmetic Ingredients) publié par le CTFA (Cosmetic, Toiletry and Fragrance Association).

La composition cosmétique ou dermatologique selon la présente invention peut être, par exemple une composition pour le soin du visage, du corps, par exemple des compositions pour le visage et/ou le corps.

Selon l'invention la composition cosmétique ou dermatologique peut être une crème solaire ou écran solaire.

Selon l'invention, la composition selon la présente invention peut être une composition pour application topique.

La présente invention se rapporte également à un procédé de fabrication d'une composition selon l'invention, ledit procédé comprenant une étape de mélange du diéthylamino hydoxybenzoyl hexyl benzoate, du méthylene bis-benzotriazolyl tetramethylbutylphenol, de l'éthylhexyl triazone, du bis-ethylhexyloxyphenol methoxyphenyl triazine et du dioxide de titane ($TiO_2$) une composition cosmétique ou dermatologique.

La composition cosmétique ou dermatologique de l'invention peut être obtenue par tout procédé approprié connu de l'homme du métier pour la fabrication d'une composition cosmétique et/ou dermatologique. Il peut s'agir, par exemple d'un simple mélange. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), procédé classiquement utilisé par l'homme de l'art pour obtenir des émulsions huile dans eau dont les gouttelettes dispersées sont particulièrement fines, par exemple avec un diamètre de 0,1 à 1 $\mu$m.

Les inventeurs ont démontré de manière surprenante que la composition selon l'invention comprenant du diéthylamino hydoxybenzoyl hexyl benzoate, du méthylene bis-benzotriazolyl tetramethylbutylphenol, de l'éthylhexyl triazone, du bis-ethylhexyloxyphenol methoxyphenyl triazine et du dioxide de titane (TiO$_2$) permet de filtrer les rayonnements UVA, UVB et les rayonnements visibles de forte énergie. Aussi, la composition selon l'invention permet avantageusement, après application topique une protection de la peau contre les rayonnements Ultraviolets (U.V.) et la lumière visible de haute énergie.

En outre les inventeurs ont démontré de manière surprenante que la composition selon l'invention peut être avantageusement transparente et permet de filtrer les rayonnements UVA, UVB et les rayonnements visibles de haute énergie.

Avantageusement de part le filtrage/blocage des UVA, UVB et des rayonnements visibles de hautes énergies la composition selon l'invention peut être avantageusement utilisée par des personnes présentant une hyperpigmentation cutanée, par exemple un mélasma, afin de protéger leur peau, à la fois des UVA, UVB et des rayonnements visibles de hautes énergies permettant ainsi avantageusement de limiter la pigmentation due à ces rayons et/ou l'apparition de nouvelles taches cutanées d'hyperpigmentation.

La présente invention a également pour objet l'utilisation cosmétique non thérapeutique de la composition selon l'invention pour la protection de la peau contre les rayonnements Ultra-Violets (U.V.) et la lumière visible de haute énergie.

La présente invention a également pour la composition selon l'invention pour son utilisation pour la protection de la peau contre les rayonnements Ultra-Violets (U.V.) et la lumière visible de haute énergie.

La présente invention a également pour la composition selon l'invention pour son utilisation pour la protection de la peau contre les rayonnements Ultra-Violets (U.V.) de longueur d'onde comprise de 280 à 400nm et la lumière visible de haute énergie lumière visible de haute énergie de longueur d'onde comprise de 380 à 483nm.

La présente invention a également pour objet la composition selon l'invention pour son utilisation pour la protection de la peau contre les rayonnements UVA, UVB et la lumière visible avec une longueur d'onde de 380-483nm comprenant le champs chromatique violet 380-449 nm, violet-bleu 449-466nm, bleu-violet 466-478 nm et Bleu 478-483nm.

La présente invention a également pour objet l'utilisation cosmétique non thérapeutique de la composition selon l'invention pour la protection de la peau contre les rayonnements UVA, UVB et la lumière visible avec une longueur d'onde de 380-483nm comprenant le champs chromatique violet 380-449 nm, violet-bleu 449-466nm, bleu-violet 466-478 nm et Bleu 478-483nm.

La présente invention a également pour objet une composition selon l'invention pour son utilisation dans la prévention et/ou le traitement du mélasma et/ou de l'hyperpigmentation cutanée et/ou des taches pigmentaires de la peau et/ou des taches cutanées d'hyperpigmentation de la peau.

Dans la présente par prévention du mélasma on entend l'absence et/ou une diminution de l'apparition de taches pigmentaires sur la peau, par exemple sur la peau de femmes enceinte.

Dans la présente par traitement du mélasma on entend une réduction et/ou une diminution de la tailles et/ou du nombre de taches pigmentaires pouvant allez jusqu'à une disparation totale des taches pigmentaires.

Dans la présente par traitement l'hyperpigmentation cutanée et/ou des taches pigmentaires de la peau et/ou des taches cutanées d'hyperpigmentation de la peau on entend une réduction et/ou une diminution de la tailles et/ou du nombre de taches pigmentaires pouvant allez jusqu'à une disparation totale des taches pigmentaires.

Dans la présente par prévention de l'hyperpigmentation cutanée et/ou des taches pigmentaires de la peau et/ou des taches cutanées d'hyperpigmentation de la peau on entend l'absence et/ou une diminution de l'apparition de taches pigmentaires sur la peau, par exemple sur la peau de femmes enceinte.

Dans la présente par traitement du mélasma on entend une réduction et/ou une diminution de la tailles et/ou du nombre de taches pigmentaires pouvant allez jusqu'à une disparation totale des taches pigmentaires.

La présente invention a également pour objet une composition selon l'invention pour son utilisation dans la prévention et/ou la prophylaxie et/ou dans le traitement de mélanome.

La présente invention a également pour objet l'utilisation cosmétique non thérapeutique de la composition selon l'invention pour la protection de la peau contre les rayonnements UVA, UVB et la lumière visible bleue et violette.

La présente invention a également pour objet la composition selon l'invention pour son utilisation pour la protection de la peau contre les rayonnements et la lumière visible pigmentogènes.

La présente invention a également pour objet l'utilisation cosmétique non thérapeutique de la composition selon l'invention pour la protection de la peau contre les rayonnements et la lumière visible pigmentogènes.

Pour utilisation, on peut, par exemple, utiliser les formes galéniques décrites ci-dessus. L'utilisation peut donc être réalisée en fonction de la forme galénique utilisé.

La présente invention a également pour objet l'utilisation de la composition selon l'invention comme écran solaire.

La présente invention a également pour objet la composition selon l'invention pour son utilisation comme écran solaire, par exemple après application de la composition sur la peau.

Pour cette utilisation, on peut, par exemple, utiliser les formes galéniques décrites ci-dessus. L'utilisation peut donc être réalisée en fonction de la forme galénique utilisée.

La présente invention a également pour objet un procédé de traitement cosmétique non thérapeutique comprenant l'application sur la peau d'une composition cosmétique selon l'invention.

Selon l'invention, le traitement cosmétique peut-être un traitement pour protéger la peau des rayonnements Ultraviolets (U.V.) et la lumière visible de haute énergie.

Pour cette application, on peut, par exemple, utiliser les formes galéniques décrites ci-dessus. L'application sur la peau peut donc être réalisée en fonction de la forme galénique utilisée.

L'application peut être accompagnée, par exemple, d'un massage de la peau avec une composition selon la présente invention.

L'application peut être réalisée avec une quantité suffisante de la composition afin que toute la surface de la peau à traiter soit traitée. Il peut s'agir par exemple d'une application classique, comme une crème sur la peau.

L'application peut être toute application souhaitée par l'utilisateur/l'utilisatrice, par exemple une application quotidienne, biquotidienne ou tous les 2 jours. Il peut s'agir par exemple d'une application une fois par jour, deux fois par jour ou plus.

La présente invention se rapporte également à une trousse de soin cosmétique comprenant une composition cosmétique selon la présente invention et un manuel ou une notice d'utilisation. La trousse peut être par exemple un conditionnement.

Le manuel ou cette notice peut avoir notamment pour fonction d'expliquer à l'utilisateur la manière, la quantité de produit à appliquer et/ou la fréquence d'application sur la peau de la composition de la présente invention.

Selon l'invention, la trousse de soin cosmétique peut également comprendre un applicateur, par exemple une spatule, une brosse, une pompe, un spray.

D'autres avantages pourront encore apparaître à l'homme du métier à la lecture des exemples ci-dessous, illustrés par les figures annexées, donnés à titre illustratif.

## Brève description des figures

La figure 1 représente un diagramme de l'absorbance (DO) de rayonnement de longueur d'onde comprise de 290 à 400 nm. L'ordonné représente l'absorbance et l'abscisse la longueur d'onde.

La figure 2 représente un diagramme de l'absorption des rayonnements en fonction de la longueur d'onde. L'ordonné représente l'absorption et l'abscisse la longueur d'onde.

La figure 3 représente un diagramme de la transmission des rayonnements en fonction de la longueur d'onde. L'ordonné représente le pourcentage de transmission et l'abscisse la longueur d'onde.

## EXEMPLES

### Exemple 1 : Composition cosmétique ou dermatologique contre les rayonnements UV et la lumière visible de haute énergie

Les ingrédients provenaient indépendamment des sociétés BASF, Croda, Azelis.

Dans cet exemple, un exemple de composition selon l'invention a été préparée selon le procédé suivant utilisant un Emulseur (Machine Dumek 10Kg) référencé (E) ci-dessous avec une vitesse de rotation à 100% de 2850 tours par minute et un agitateur Planétaire et contre agitation (Machine Dumek 10Kg) référencé (P) ci-dessous avec une vitesse d'agitation à 100% de 28,5 tours par minute. Lors de ce procédé différentes phases de la composition ont été préparées et mélangées comme suit :

Phase A : Ouvrir la cuve, sans agitation, peser l'eau, toujours cuve ouverte, sous E 30% l'EDETA, le pentylene glycol et le potassium sorbate ont été ajoutés puis la cuve fermée et l'émulseur stoppé. Une homogénéisation a été réalisée sous agitation P100% et un chauffage à 50°C commencé.

Phase B : un prémélange Xanthan dans la glycérine a été préparé puis à 50°C, en cuve ouverte, sous E30%, la phase B a été ajoutée dans la phase A. La cuve a été refermée et l'émulseur stoppé. Puis l'ensemble a été agité sous P100% jusqu'à l'obtention d'une phase homogène.

Phase A' : Puis à 80°C, cuve ouverte, sous E30%, de la chlorphénesin BP73 commercialisé par la société azelis a été ajoutée. La cuve a été refermée et l'émulseur stoppé. L'ensemble a été ensuite mis sous agitation sous P100. Une phase C a été indépendamment préparée comme suit : les composants de la phase C, à savoir les emulgateurs, uvinul A plus granular, le solubilisant, uvinul T150, le solaveil XT300-LQ-WD et la pearlpurine PP ont été pesés puis mis sous agitation et chauffé à 80°C jusqu'à l'obtention d'une phase homogène. A 80°C, sous agitation P100% et E100% + vide -350mbar, la phase C a été introduite dans la phase A+B+A' par l'entonnoir en filet (Temps d'introduction de la phase C = 8 minutes puis agité pendant 12 min.). Un refroidissement par paliers, à savoir tous les 5°C a été réalisé sous agitation P100%.

Une phase D a été préparée comme suit : un prémélange tinosorb dans de l'eau a été préparé puis à 60°C sous agitation P100%, la phase D a été ajoutée dans la phase principale par l'entonnoir en filet via un temps d'introduction de 5 min et temps d'agitation de 10 min puis refroidissement sous P100%.

Une phase E a été préparée à 50°C, par l'entonnoir, sous agitation P100%, via l'ajout de phenoxetol. Puis l'ensemble a été laissé agité jusqu'à l'obtention d'une phase homogène. Du cyclopentasiloxane a été ajouté par l'entonnoir en filet sous P100% + E30% pendant E = 2 min. Le refroidissement a été continué sous P100% et commencée à faire le vide à plusieurs reprises. (-950mbar).

Une Phase F a été préparée par agitation via la dispersion de xylitol dans l'eau et agitation jusqu'à l'obtention d'une phase homogène. A 35°C, par l'entonnoir, sous agitation P100%, la phase F a été ajouté au mélange puis le vide a été effectué à -950mbar.

Une Phase G a été préparée par dispersion sous agitation, de l'AA2G dans l'eau puis le pH a été ajusté avec de la soude solution aqueuse à 10% (pH de la phase environ 5,2). A 35°C, par l'entonnoir, sous agitation P100%, la phase G a été ajoutée puis le vide a été effectué à - 950mbar.

Une Phase H a été introduite comme suit à 35°C, la cuve a été ouverte et une solution d'acide citrique à 50 % a été ajouté puis la cuve refermée et laissée agiter sous P100%.

Enfin en cuve ouverte, de la vitamine E a été ajoutée, la cuve refermée et un vide effectuée à -950 mbar et agiter jusqu'à l'obtention d'une phase homogène.

Le pH a été vérifiée et l'ajusté entre 5,8 <pH <6,2 si nécessaire avec la solution d'acide citrique à 50%.

Ainsi, la composition décrite dans le tableau 1 ci-dessous a été préparée et obtenue.

Tableau 1 : composition anti-UV/visible ou écran solaire filtrant les UV et la lumière visible de haute énergie.

| Ingrédients | Pourcentage en poids par rapport au poids total |
|---|---|
| Eau | 25-50 |
| Diisopropyl Sebacate | 10-25 |
| Diethylamino Hydroxybenzoyl Hexyl Benzoate | 5-10 |
| Bis-Ethylhexyloxyphenol Methoxyphenyl Triazine | 5-10 |
| Ethylhexyl Triazone | 1-5 |
| Methylene Bis-Benzotriazolyl Tetramethylbutylphenol [nano] | 1-5 |
| Glycerin | 1-5 |
| CI 77891 titanium dioxide | 1-5 |
| Caprylic/Capric Triglycéride | 1-5 |
| Potassium Cetyl Phosphate | 1-5 |
| Cyclopentasiloxane | 1-5 |
| Glyceryl stearate | 1-5 |
| PEG-100 Stearate | 1-5 |
| Pentylene Glycol | 1-5 |
| Coco-Glucoside | 0,1-1 |
| Polyhydroxystearic Acid | 0,1-1 |
| Decyl Glucoside | 0,1-1 |
| Phenoxyethanol | 0,1-1 |
| Xylitol | 0,1-1 |
| Coconut Alcohol | 0,1-1 |
| Alumina | 0,1-1 |

(suite)

| Ingrédients | Pourcentage en poids par rapport au poids total |
|---|---|
| Stearic Acid | 0,1-1 |
| Chlorphenesin | 0,1-1 |
| Xanthan Gum | 0,1-1 |
| Disodium EDTA | 0,1-1 |
| Potassium sorbate | 0,1-1 |
| Pearl Powder | 0,1-1 |
| Ascorbyl Glucoside | 0,1-1 |
| Tocopheryl Acetate | 0,1-1 |
| Citric Acid | 0-0,1 |
| Propylene glycol | 0-0,1 |
| Sodium Hydroxide | 0-0,1 |

**Exemple 2 : évaluation de la filtration des rayonnements UV et de la lumière visible par une composition selon l'invention**

Dans le présent exemple la composition utilisée était la suivante :

Tableau 2 : composition anti-UV / lumière visible de haute énergie ou écran solaire filtrant les UV et la lumière visible de haute énergie.

| Ingrédients | Pourcentage en poids par rapport au poids total |
|---|---|
| Eau | 25-50 |
| Diisopropyl Sebacate | 10-25 |
| Diethylamino Hydroxybenzoyl Hexyl Benzoate | 7-9 |
| Bis-Ethylhexyloxyphenol Methoxyphenyl Triazine | 4-6 |
| Ethylhexyl Triazone | 3-5 |
| Methylene Bis-Benzotriazolyl Tetramethylbutylphenol [nano] | 4-5 |
| Glycerin | 1-5 |
| CI 77891 titanium dioxide | 3-4 |
| Caprylic/Capric Triglycéride | 1-5 |
| Potassium Cetyl Phosphate | 1-5 |
| Cyclopentasiloxane | 1-5 |
| Glyceryl stearate | 1-5 |
| PEG-100 Stearate | 1-5 |
| Pentylene Glycol | 1-5 |
| Coco-Glucoside | 0,1-1 |
| Polyhydroxystearic Acid | 0,1-1 |
| Decyl Glucoside | 0,1-1 |
| Phenoxyethanol | 0,1-1 |
| Xylitol | 0,1-1 |

(suite)

| Ingrédients | Pourcentage en poids par rapport au poids total |
|---|---|
| Coconut Alcohol | 0,1-1 |
| Alumina | 0,1-1 |
| Stearic Acid | 0,1-1 |
| Chlorphenesin | 0,1-1 |
| Xanthan Gum | 0,1-1 |
| Disodium EDTA | 0,1-1 |
| Potassium sorbate | 0,1-1 |
| Pearl Powder | 0,1-1 |
| Ascorbyl Glucoside | 0,1-1 |
| Tocopheryl Acetate | 0,1-1 |
| Citric Acid | 0-0,1 |
| Propylene glycol | 0-0,1 |
| Sodium Hydroxide | 0-0,1 |

Dans le présent exemple une évaluation de la largeur de protection d'un écran solaire /composition selon l'invention sur la totalité du spectre UV par la détermination des longueurs d'onde à laquelle 90% de l'absorbance globale est atteinte.

Une quantité appropriée de produit a été appliqué sur le substrat permettant la mesure de l'absorption dans la gamme des UV. La superficie totale sous la courbe d'absorbance obtenue étant égale à 100%, la longueur d'onde critique a été déterminée mathématiquement à partir de cette courbe que la valeur de la longueur d'onde à laquelle l'aire sous la courbe atteint 90% de la superficie totale, lorsque l'on part de 290 nm.

Il a été établi que cette valeur doit être égale ou supérieure à 370 nm de manière à classer le produit comme à large spectre.

Dans le présent exemple, un spectrophotomètre "KONTRON 933" équipée avec une source UV et un monochromateur capable de fournir un flux d'énergie entre 290 et 400 nm a été utilisé. Une balance de laboratoire de précision pour contrôler le poids de produit déposé a été utilise et la composition a été déposée avec une spatule.

Suntest CPS ou CPS + (ATLAS) avec filtre standard ou filtre «Colipa» a été utilisé. Une régulation de l'équipement dans la gamme de température de 25 à 35 °C a été assurée par l'utilisation de la climatisation. Le spectre de la source d'UV artificiel est aussi semblable que possible à un soleil standard défini par le Colipa (1994) ou dans le DIN67501 (1996).

Le produit a été appliqué sur des plaques de PMMA. Plaques PMMA de rugosité spécifiée et la taille sont déjà utilisés dans de nombreuses parties du monde et sont recommandé par le Colipa.

Les plaques ont été rendues rugueuses d'un côté afin d'obtenir une surface rugueuse tridimensionnel pour mesure de la topographie de la surface (Sa) entre 4 et 6 micromètres. Les valeurs Sa sont reconnues par des études de validation et sont comparables à celles recommandées par le Colipa directrice Mars 2011, Final Draft (incorporant des Plaques 6 μm et la norme S2).

Les expériences ont été réalisées comme suit :

Dépôt de la composition sur le support : le dépôt a été effectué comme de petits spots alignés partout sur la surface. Une fois déposée, la quantité de produit a été vérifiée par pesée. Taux d'application a été déterminé de telle sorte que la quantité réelle de produit laissé sur le substrat avant équilibration était 0,75 mg / cm2 pour la plaque PMMA.

Un étalement de la composition a été effectué afin d'obtenir un film homogène le plus possible, avec propagation très légère pendant environ 30 secondes suivi par la diffusion avec une plus grande pression pendant environ 30 secondes.

Irradiation UV : dans ce test, a été utilisé un Suntests CPS +, ATLAS, avec une délivrance avec pré-irradiation de 4 fois 200 J / m2-efficace (eff) (800 J / m2-EFF).

Les mesures de transmission des UV à travers la plaque comprenant la composition a été réalisée par disposition dans le parcours de lumière de la source d'UV et du dispositif de mesure de la valeur moyenne de transmission du rayonnement UV à travers l'échantillon (En utilisant les données d'absorbance monochromatique mesurées sur différents sites au sein de la plaque ou par la mesure de l'ensemble plaque) déterminée à 290-400 nm

Chaque plaque a été mesurée sur différent site et ainsi 5 mesures ont été réalisées sur 3 plaques (21 CFR 201,327 (j)).

La mesure de la valeur critique de rayonnement a été déterminée selon la formule suivante :

$$\int_{290\,nm}^{\lambda c} a(\lambda) \times \delta(\lambda) = 0.9 \times \int_{290\,nm}^{400\,nm} a(\lambda) \times \delta(\lambda)$$

dans lesquelles a(λ) est l'Absorbance,

λc est la longueur d'onde à laquelle la courbe d'absorbance atteint 90% de l'aire total entre 290 nm and 400 nm.

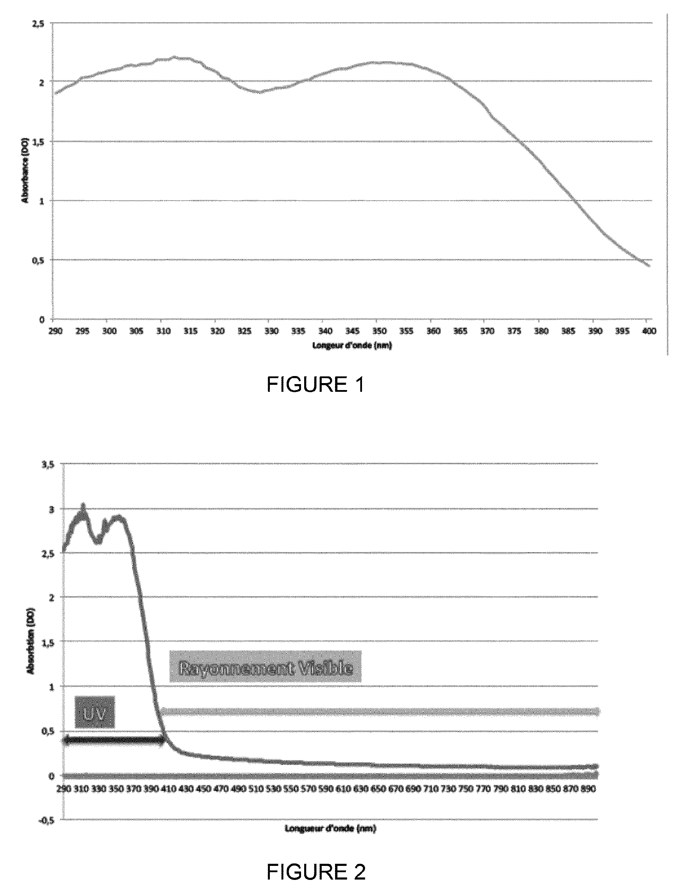

Après mesure (données non fournies) et tel qu'illustré sur la figure 1, il a été démontrée que la composition selon l'invention à une valeur λc de 378 nm.

En particulier, la figure 1 démontre clairement que la composition selon l'invention présente une absorption des rayonnements de longueurs d'onde de 200 à 398 nm

Une évaluation du facteur de protection solaire, dit « sun protection factor » ou S.P.F, par une méthode spectrophotométrique, initialement décrite par B.L. Diffey et J Robson (J.S.C.C. 40, 127-133 May/June 1989) a été également effectué.

Pour déterminer l'efficacité des produits solaires, on évalue leur capacité à prévenir l'érythème qui apparaît à la surface de la peau si celle-ci est exposée un certain temps à une source d'énergie UV. Les UVB représentent environ 5 % de l'énergie totale des UV reçus (UVA + UVB).

La méthode utilisée consiste à mesurer le flux d'énergie UV traversant la crème, exprimé en transmission énergétique et à comparer ce flux au flux initial selon le principe de toute méthode spectrophotométrique.

$$F(\lambda) = I / I0 \text{ ou } \lambda \text{ est la longueur d'onde}.$$

ou λ est la longueur d'onde.

Toutefois, la comparaison de ces deux courbes n'est pas suffisante à exprimer le niveau de protection du produit étalé sur la peau puisque celle-ci va dépendre de deux autres fonctions d'onde

Celle concernant la source : dans la pratique, il s'agit du soleil dont le spectre a été défini par la commission internationale de l'énergie comme une fonction d'onde G(λ)

Celle concernant la peau : les réactions cutanées ou sous cutanées dépendent bien entendu de l'énergie de la lumière d'excitation et une courbe réponse, sous forme d'une fonction d'onde H(λ)

Selon les mêmes principes que la colorimétrie, une courbe résultante à partir de l'intégration du produit des trois courbes est exprimée.

En intégrant chacune de ces 3 courbes sur tout le domaine UVB et UVA et en faisant le produit des 3 intégrales, l'expression du SPF In Vitro est obtenu.

Cette méthode a été initialement décrite par B Diffey et J Robson (JSCC 40. 127-133 (1989). Elle est aujourd'hui largement utilisée et reconnue par les instances internationales.

Pour ce faire un spectrophotomètre UV Kontron, équipé d'une source UV et d'un monochromateur, capable de délivrer un flux d'énergie UV entre 290 et 400 nm a été utilisé.

Une balance de laboratoire de précision pour contrôler les pesées.

Les crèmes ont été déposées avec des spatules de laboratoire ou une micro pipette MICROMAN de 250 ml GILSON avec capillaire et piston.

La composition a été étalé les produits suivant la procédure décrite plus bas sur les supports suivants :

• Bandes TRANSPORE de la société 3M

• Plaques de PMMA

La composition a été étalée suivant la quantité préconisée soit 2 mg/cm$^2$ pesé avec précision avant étalement, pour le Transpore et 1,2 mg/cm$^2$ pour le PMMA.

La composition a été étalée afin de réaliser un film le plus homogène possible.

Une calibration a été préalablement calibré l'appareil de mesures avec des plaques PMMA de références, pour s'assurer de la bonne marche de celui-ci, afin d'assurer la qualité de nos résultats.

Préalablement aux mesures, une irradiation des plaques, sur lesquelles la crème avait été étalée a été réalisé, dans un Suntest Atlas CPS+ ; qui reproduit la lumière solaire. Les plaques ont été placées dans le Suntest et une dose de 2 DEM est délivrée en 1/2 heure (550 Watt/m$^2$)

Le calcul du SPF in-vitro a été réalisé à partir de l'ensemble du spectre résiduel UVB et UVA ayant traversé la couche de crème étalée sur la plaque. Toutefois, cette fonction d'onde T($\lambda$) a été multipliée par une première fonction d'onde qui exprime la caractéristique spectrale du soleil. S($\lambda$) et une seconde fonction d'onde qui exprime la réactivité de la peau en fonction de la longueur d'onde (et donc de l'énergie dissipée) : c'est la fonction érythémateuse E($\lambda$) selon la formule ci-dessous

$$SPF\ in\ vitro = \frac{\displaystyle\sum_{290\,nm}^{400nm} E(\lambda).S(\lambda).d\lambda}{\displaystyle\sum_{290\,nm}^{400nm} E(\lambda).S(\lambda).T(\lambda).d\lambda}$$

La protection UVA IN VITRO s'exprime à partir de l'ensemble du spectre résiduel UVA ayant traversé la même couche de crème.

$$UVA_e\ in\ vitro = \frac{\displaystyle\sum_{320\,nm}^{400nm} E(\lambda).S(\lambda).d\lambda}{\displaystyle\sum_{320\,nm}^{400nm} E(\lambda).S(\lambda).T(\lambda).d\lambda}$$

Les résultats obtenus sont résumés dans le tableau 4 ci-dessous :

|         | SPF   | UVA  |
|---------|-------|------|
| Série 1 | 91,3  | 41,6 |
| Série 2 | 98,1  | 50,0 |
| Série 3 | 119,4 | 51,6 |

(suite)

|  | SPF | UVA |
|---|---|---|
| Série 4 | 107,6 | 46,6 |
| Moyenne | 107,6 | 46,6 |
| Ecart type | 15,1 | 7,2 |

Le ratio SPF/UVA est égale à : 2,30.

Ainsi, tel que démontré dans cet exemple, une composition selon l'invention présente des valeurs calculées de SPF = 107,6 et UVA = 46,6 avec un ratio SPF/UVA : SPF/UVA = 2,30, Ratio Corrigé (<3) : 60/46,6 = 1,28.

Tel que démontré dans cet exemple la composition selon l'invention présente un SPF très important et donc en accord avec la mention 50+ et protégeant des UVA et UVB.

Une évaluation de la composition concernant les rayonnements de la lumière visible a été également réalisée. Pour ce faire, une étude a été réalisée en effectuant une mesure spectrophotométrique après étalement sur plaques de PMMA.

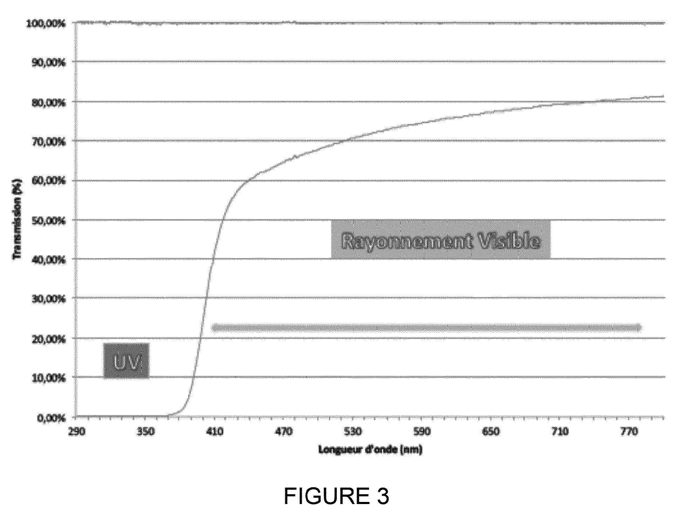

Pour ce faire le procédé utilisé était identique à celui mentionné ci-dessus sauf que le balayage du spectre se situait entre 800nm et 290nm Les résultats obtenus sont représentés sur les figures 2 et 3.

Tel que démontrer, une composition selon l'invention permet avantageusement de filtrer à la fois les rayonnements UVA et UVB et également les rayonnements de hautes énergie de la lumière visible (du bleu au violet).

## Revendications

1. Composition cosmétique et/ou dermatologique contre les rayonnements UV et la lumière visible de haute énergie comprenant du diéthylamino hydoxybenzoyl hexyl benzoate, du méthylene bis-benzotriazolyl tetramethylbutylphenol, de l'éthylhexyl triazone, du bis-ethylhexyloxyphenol methoxyphenyl triazine et du dioxide de titane ($TiO_2$).

2. Composition selon la revendication 1, dans laquelle la concentration de diéthylamino hydoxybenzoyl hexyl benzoate est de 5% à 10% en poids par rapport au poids total de la composition.

3. Composition selon la revendication 1 ou 2, dans laquelle la concentration de Bis-ethylhexyloxyphénol methoxyphenyl triazine est de 5% à 10% en poids par rapport au poids total de la composition.

4. Composition selon l'une quelconque des revendications précédentes, dans laquelle la concentration d'éthylhexyl triazone est de 1% à 5% en poids par rapport au poids total de la composition.

5. Composition selon l'une quelconque des revendications précédentes, dans laquelle la concentration de méthylène bis-benzotraizolyl est de 1% à 5% en poids par rapport au poids total de la composition.

6. Composition selon l'une quelconque des revendications précédentes, dans laquelle la concentration de dioxide de titane est de 1% à 5% en poids par rapport au poids total de la composition.

7. Composition selon l'une quelconque des revendications précédentes, ladite composition étant sous l'une des formes choisies parmi une crème, un lait, un gel, un masque.

8. Composition selon l'une quelconque des revendications précédentes, ladite composition est une crème solaire.

9. Composition selon l'une quelconque des revendications 1 à 8 pour son utilisation pour la protection de la peau contre les rayonnements UltraViolet (U.V.) et la lumière visible de haute énergie.

10. Composition selon l'une quelconque des revendications 1 à 8 pour son utilisation pour la protection de la peau contre les rayonnements UltraViolet (U.V.) de longueur d'onde comprise de 280 à 400nm et la lumière visible de haute énergie de longueur d'onde comprise de 380 à 483nm.

FIGURE 1

FIGURE 2

FIGURE 3

Europäisches
Patentamt

European
Patent Office

Office européen
des brevets

## RAPPORT DE RECHERCHE EUROPEENNE

Numéro de la demande

EP 16 17 2282

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | DATABASE GNPD [Online] MINTEL; 30 avril 2012 (2012-04-30), "Ultra UV Defense Sun Cream for the Face SPF 50+", XP002745474, Database accession no. 1770734 * Product Description * * Inner Pack Information (page 2). * ----- | 1,2,4-10 | INV. A61K8/29 A61K8/41 A61K8/49 A61Q17/04 |
| X | DATABASE GNPD [Online] MINTEL; 30 juin 2014 (2014-06-30), "Cell Protector SPF 50+ PA++", XP002745475, Database accession no. 2456775 * Product Desction * * Ingredients * ----- | 1,7-10 | |
| X | DATABASE GNPD [Online] MINTEL; 31 octobre 2014 (2014-10-31), "Protective Gel Cream SPF 60", XP002745476, Database accession no. 2753815 * Ingredients * ----- | 1,7-10 | DOMAINES TECHNIQUES RECHERCHES (IPC) A61K A61Q |
| A | WO 2011/112414 A2 (LIPO CHEMICALS INC [US]; PHOTOPROTECTIVE TECHN INC [US]; DAYAN NAVA [U) 15 septembre 2011 (2011-09-15) * alinéa [0001] * * alinéa [0014] * * alinéa [0016] - alinéa [0022] * * alinéa [0028] * * alinéa [0072] * ----- -/-- | 1-10 | |

Le présent rapport a été établi pour toutes les revendications

| Lieu de la recherche | Date d'achèvement de la recherche | Examinateur |
|---|---|---|
| Munich | 13 juillet 2016 | Irwin, Lucy |

**Europäisches Patentamt**
**European Patent Office**
**Office européen des brevets**

# RAPPORT DE RECHERCHE EUROPEENNE

Numéro de la demande

EP 16 17 2282

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | FR 2 903 599 A1 (L'OREAL) 18 janvier 2008 (2008-01-18) * page 1 * * page 12 - page 14 * * revendications 1,22-26 * ----- | 1-10 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 13 juillet 2016 | Irwin, Lucy |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 16 17 2282

13-07-2016

| Document brevet cité au rapport de recherche | | Date de publication | Membre(s) de la famille de brevet(s) | | Date de publication |
|---|---|---|---|---|---|
| WO 2011112414 | A2 | 15-09-2011 | CA | 2792811 A1 | 15-09-2011 |
| | | | CN | 103025333 A | 03-04-2013 |
| | | | EP | 2544691 A2 | 16-01-2013 |
| | | | JP | 2013522198 A | 13-06-2013 |
| | | | US | 2013078205 A1 | 28-03-2013 |
| | | | WO | 2011112414 A2 | 15-09-2011 |
| FR 2903599 | A1 | 18-01-2008 | EP | 2046275 A1 | 15-04-2009 |
| | | | FR | 2903599 A1 | 18-01-2008 |
| | | | JP | 5730482 B2 | 10-06-2015 |
| | | | JP | 2009542758 A | 03-12-2009 |
| | | | US | 2010104520 A1 | 29-04-2010 |
| | | | WO | 2008006687 A1 | 17-01-2008 |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **B.L. DIFFEY ; J ROBSON.** *J.S.C.C.,* Mai 1989, vol. 40, 127-133

- **B DIFFEY ; J ROBSON.** *JSCC,* 1989, vol. 40, 127-133